(19) Europäisches Patentamt
European Patent Office
Office européen des brevets

(11) Veröffentlichungsnummer: **0 503 338 A1**

# (12) EUROPÄISCHE PATENTANMELDUNG

(21) Anmeldenummer: **92103013.6**

(22) Anmeldetag: **22.02.92**

(51) Int. Cl.5: **C07D 235/12**, C07D 235/14, C07D 263/56, C07D 277/64, C08K 5/3447, C08K 5/353, C08K 5/47, A61K 7/42

(30) Priorität: **08.03.91 DE 4107379**

(43) Veröffentlichungstag der Anmeldung:
**16.09.92 Patentblatt 92/38**

(84) Benannte Vertragsstaaten:
**CH DE FR GB IT LI**

(71) Anmelder: **BASF Aktiengesellschaft**
**Carl-Bosch-Strasse 38**
**W-6700 Ludwigshafen(DE)**

(72) Erfinder: **Holderbaum, Martin, Dr.**
**Maudacher Strasse 366**
**W-6700 Ludwigshafen(DE)**
Erfinder: **Aumueller, Alexander, Dr.**
**Rieslingweg 25**
**W-6730 Neustadt(DE)**
Erfinder: **Trauth, Hubert**
**Milanstrasse 6**
**W-6724 Dudenhofen(DE)**

(54) **Aminovinyl-substituierte Heterocyclen als Stabilisatoren für organische Materialien.**

(57) Verwendung von Aminovinyl-substituierten Heterocyclen I

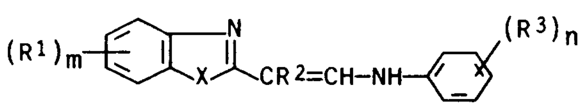

I

in der

- $R^1$ Wasserstoff, $C_1$-$C_4$-Alkyl, $C_1$-$C_4$-Alkoxy oder Halogen bezeichnet, wobei beim Vorliegen mehrerer Reste $R^1$ diese gleich oder verschieden sein können,
- $R^2$ Cyano oder $C_1$-$C_{20}$-Alkoxy- oder $C_3$-$C_6$-Cycloalkoxycarbonyl bedeutet,
- $R^3$ für Wasserstoff, $C_1$-$C_{18}$-Alkyl, $C_1$-$C_{18}$-Alkoxy, Halogen, Cyano oder $C_1$-$C_{20}$-Alkoxy- oder $C_3$-$C_6$-Cycloalkoxycarbonyl steht, wobei beim Vorliegen mehrerer Reste $R^3$ diese gleich oder verschieden sein können,
- X NH, O oder S bedeutet,
- m für die Zahl 1 oder 2 steht und
- n eine ganze Zahl von 1 bis 5 bezeichnet,

als Stabilisatoren für organische Materialien.

EP 0 503 338 A1

Die vorliegende Erfindung betrifft die Verwendung von Aminovinyl-substituierten Heterocyclen der allgemeinen Formel I

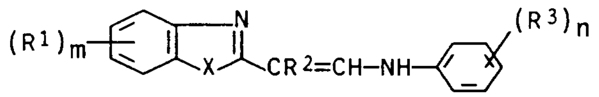

I

in der

- $R^1$ Wasserstoff, $C_1$-$C_4$-Alkyl, $C_1$-$C_4$-Alkoxy oder Halogen bezeichnet, wobei beim Vorliegen mehrerer Reste $R^1$ diese gleich oder verschieden sein können,
- $R^2$ Cyano oder $C_1$-$C_{20}$-Alkoxy- oder $C_3$-$C_6$-Cycloalkoxycarbonyl bedeutet,
- $R^3$ für Wasserstoff, $C_1$-$C_{18}$-Alkyl, $C_1$-$C_{18}$-Alkoxy, Halogen, Cyano oder $C_1$-$C_{20}$-Alkoxy- oder $C_3$-$C_6$-Cycloalkoxycarbonyl steht, wobei beim Vorliegen mehrerer Reste $R^3$ diese gleich oder verschieden sein können,
- X NH, O oder S bedeutet,
- m für die Zahl 1 oder 2 steht und
- n eine ganze Zahl von 1 bis 5 bezeichnet,

als Stabilisatoren für organische Materialien.

Ein Teil der Aminovinyl-substituierten Heterocyclen I sind neue Verbindungen.

Weiterhin betrifft die Erfindung die Verbindungen I enthaltende und somit gegen die Einwirkung von Licht, Sauerstoff und Wärme stabilisierte organische Materialien, insbesondere stabilisierte Kunststoffe und Lacke, sowie die Verbindungen I als Lichtschutzmittel enthaltende kosmetische Zubereitungen.

Organisches Material, insbesondere Kunststoffe und Lacke, wird bekanntermaßen sehr schnell, vor allem durch Einwirkung von Licht, zerstört. Diese Zerstörung zeigt sich üblicherweise in Vergilbung, Verfärbung, Rißbildung oder Versprödung des Materials. Mit den bisher verwendeten Lichtschutzmitteln und Stabilisatoren konnte kein zufriedenstellender Schutz gegen die Zerstörung von organischem Material durch Licht, Sauerstoff und Wärme erzielt werden.

So werden beispielsweise in der US-A 3 079 366 (1) u.a. Arylaminoethylene der Formel IV

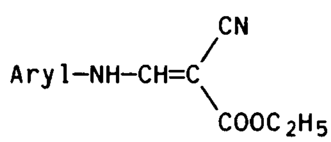

IV

als UV-Absorber für Kunststoffe empfohlen. Die Verbindungen IV weisen zwar die gewünschten spektroskopischen Eigenschaften auf, genügen jedoch hinsichtlich ihrer Stabilisierungs- bzw. Lichtschutzwirkung nicht den heute gestellten Anforderungen. Insbesondere ist die Vergilbungsneigung der mit den Verbindungen IV stabilisierten Kunststoffe immer noch zu hoch.

Aufgabe der vorliegenden Erfindung war es daher, Lichtschutzmittel bzw. Stabilisatoren bereitzustellen, die einen wirkungsvollen Schutz für organisches Material mit sich bringen.

Demgemäß wurde die eingangs definierte Verwendung der Aminovinyl-substituierten Heterocyclen I gefunden.

Außer Wasserstoff bezeichnet $R^1$ $C_1$-$C_4$-Alkyl wie Methyl, Ethyl, n-Propyl, iso-Propyl, n-Butyl, iso-Butyl, sec.-Butyl oder tert.-Butyl, $C_1$-$C_4$-Alkoxy wie Methoxy, Ethoxy, n-Propoxy, iso-Propoxy, n-Butoxy, iso-Butoxy, sec.-Butoxy oder tert.-Butoxy, sowie Halogen wie Fluor, Chlor, Chrom oder Iod. Bevorzugt werden hiervon Wasserstoff, Methyl, Methoxy und Chlor, insbesondere Wasserstoff allein. Die Reste $R^1$ befinden sich vornehmlich in der 5- und/oder 6-Position des Benzimidazol-Systems.

Der Rest $R^2$ bedeutet Cyano oder vorzugsweise Alkoxycarbonyl, wobei der Alkylteil dieser Carbonsäurealkylester-Funktion vor allem aus einer $C_1$-$C_{20}$-Alkylgruppe, daneben aber auch aus einer $C_3$-$C_6$-Cycloalkylgruppe bestehen kann. Als geradkettige oder verzweigte $C_1$-$C_{20}$-Alkylgruppen eignen sich hierbei vor allem Methyl, Ethyl, n-Propyl, iso-Propyl, n-Butyl, iso-Butyl, sec.-Butyl, tert.-Butyl, n-Pentyl, n-Hexyl, n-Heptyl, n-Octyl, 2-Ethylhexyl, n-Nonyl, iso-Nonyl, n-Decyl, iso-Decyl, n-Undecyl, n-Dodecyl, n-Tridecyl, iso-Tridecyl, n-Tetradecyl, n-Pentadecyl, n-Hexadecyl, n-Heptadecyl, n-Octadecyl und n-Eicosyl. Die längerkettigen Alkylreste können synthetischen Ursprungs sein, beispielsweise durch Oxo- oder Ziegler-Synthese hergestellt, oder aus natürlich vorkommenden Fettalkoholen stammen. Als $C_3$-$C_6$-Cycloalkylgrup-

pen sind Cyclopropyl, Cyclobutyl, Cyclopentyl und insbesondere Cyclohexyl zu nennen.

Die Substituenten $R^3$ am Phenylkern stehen neben Wasserstoff für geradkettiges oder verzweigtes $C_1$-$C_{18}$-Alkyl wie vor allem $C_1$-$C_4$-Alkyl, z.B. n-Propyl, iso-Propyl, n-Butyl, iso-Butyl, sec.-Butyl, tert.-Butyl, insbesondere jedoch Methyl oder Ethyl, daneben aber auch n-Pentyl, n-Hexyl, n-Heptyl, n-Octyl, 2-Ethylhexyl, n-Nonyl, iso-Nonyl, n-Decyl, iso-Decyl, n-Undecyl, n-Dodecyl, n-Tridecyl, iso-Tridecyl, n-Tetradecyl, n-Pentadecyl, n-Hexadecyl, n-Heptadecyl oder n-Octadecyl. Die längerkettigen Alkylreste können synthetischen Ursprungs sein, beispielsweise durch Oxo-oder Ziegler-Synthese hergestellt, oder aus natürlich vorkommenden Fettalkoholen stammen.

Weiterhin stehen die Substituenten $R^3$ neben Wasserstoff für geradkettiges oder verzweigtes $C_1$-$C_{18}$-Alkoxy, wie vor allem $C_1$-$C_4$-Alkoxy, z.B. n-Propoxy, iso-Propoxy, n-Butoxy, iso-Butoxy, sec.-Butoxy, tert.-Butoxy, insbesondere jedoch Methoxy oder Ethoxy, daneben aber auch n-Pentoxy, n-Hexoxy, n-Heptoxy, n-Octoxy, 2-Ethylhexoxy, n-Nonoxy, iso-Nonoxy, n-Decyloxy, iso-Decyloxy, n-Undecyloxy, n-Dodecyloxy, n-Tridecyloxy, iso-Tridecyloxy, n-Tetradecyloxy, n-Pentadecyloxy, n-Hexadecyloxy, n-Heptadecyloxy oder n-Octadecyloxy. Die längerkettigen Alkoxyreste können synthetischen Ursprungs sein, beispielsweise durch Oxo- oder Ziegler-Synthese hergestellt, oder aus natürlich vorkommenden Fettalkoholen stammen.

Weiterhin stehen die Substituenten $R^3$ neben Wasserstoff für Halogen wie Fluor, Brom, Iod oder vor allem Chlor, für Cyano und/oder für Alkoxycarbonyl, wobei der Alkylteil dieser Carbonsäurealkylester-Funktion vor allem aus einer $C_1$-$C_{20}$-Alkylgruppe, vorzugsweise einer $C_1$-$C_{12}$-Alkylgruppe, insbesondere einer $C_1$-$C_8$-Alkylgruppe, daneben aber auch aus einer $C_3$-$C_6$-Cycloalkylgruppe bestehen kann. Beispiele für derartige $C_1$-$C_{20}$-Alkyl- bzw. $C_3$-$C_6$-Cycloalkylgruppen sind bei der Beschreibung der Bedeutung des Restes $R^2$ aufgeführt.

Die Anzahl n der Substituenten $R^3$ am Phenylkern kann bis zu 5, vorzugsweise bis zu 2 betragen.

Die Variable X steht vor allem für NH, daneben aber auch für O oder S. Im Fall von X = NH handelt es sich um Benzimidazole, im Fall von X = O um Benzoxazole und im Fall von X = S um Benzthiazole.

Bevorzugt werden als Stabilisatoren für organische Materialien 2-(Aminovinyl)benzimidazole I (X = NH), bei denen

- $R^1$ Wasserstoff, Methyl, Methoxy oder Chlor bezeichnet,
- $R^2$ Cyano oder $C_1$-$C_{20}$-Alkoxycarbonyl bedeutet,
- $R^3$ für Wasserstoff, $C_1$-$C_4$-Alkyl, $C_1$-$C_4$-Alkoxy, Chlor, Cyano oder $C_1$-$C_{12}$-Alkoxycarbonyl steht,
- m für die Zahl 1 oder 2 steht und
- n die Zahl 1 oder 2 bezeichnet.

Insbesondere werden hierfür solche 2-(Aminovinyl)benzimidazole I (X = NH) bevorzugt, bei denen

- $R^1$ Wasserstoff bezeichnet,
- $R^2$ $C_1$-$C_{20}$-Alkoxycarbonyl bedeutet,
- $R^3$ für Wasserstoff, Methyl, Ethyl, Methoxy, Ethoxy, Chlor, Cyano oder $C_1$-$C_8$-Alkoxycarbonyl steht und
- n die Zahl 1 oder 2 bezeichnet.

Ein Teil der Aminovinyl-substituierten Heterocyclen I sind neue Verbindungen. Deshalb betrifft die vorliegende Erfindung weiterhin Aminovinyl-substituierte Heterocyclen der allgemeinen Formel I

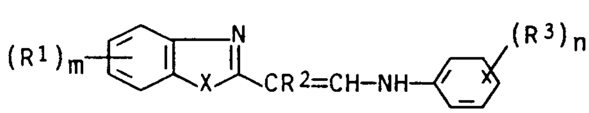

I

in der

- $R^1$ Wasserstoff, $C_1$-$C_4$-Alkyl, $C_1$-$C_4$-Alkoxy oder Halogen bezeichnet, wobei beim Vorliegen mehrerer Reste $R^1$ diese gleich oder verschieden sein können,
- $R^2$ Cyano oder $C_1$-$C_{20}$-Alkoxy- oder $C_3$-$C_6$-Cycloalkoxycarbonyl bedeutet,
- $R^3$ für Wasserstoff, $C_1$-$C_{18}$-Alkyl, $C_1$-$C_{18}$-Alkoxy, Halogen, Cyano oder $C_1$-$C_{20}$-Alkoxy- oder $C_3$-$C_6$-Cycloalkoxycarbonyl steht, wobei beim Vorliegen mehrerer Reste $R^3$ diese gleich oder verschieden sein können,
- X NH, O oder S bedeutet,
- m für die Zahl 1 oder 2 steht und
- n eine ganze Zahl von 1 bis 5 bezeichnet,

mit Ausnahme des Falles, daß gleichzeitig $R^1$ Wasserstoff bedeutet, $R^2$ Cyano bezeichnet, $R^3$ für Wasserstoff oder einen p-Methoxyrest steht und X NH bedeutet.

Vor allem betrifft die vorliegende Erfindung Aminovinyl-substituierte Heterocyclen der allgemeinen Formel I

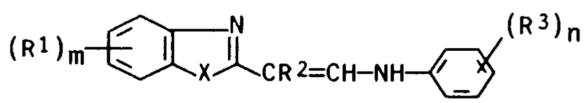

I

in der

- $R^1$ Wasserstoff, $C_1$-$C_4$-Alkyl, $C_1$-$C_4$-Alkoxy oder Halogen bezeichnet, wobei beim Vorliegen mehrerer Reste $R^1$ diese gleich oder verschieden sein können,
- $R^2$ Cyano oder $C_1$-$C_{20}$-Alkoxy- oder $C_3$-$C_6$-Cycloalkoxycarbonyl bedeutet,
- $R^3$ für Wasserstoff, $C_1$-$C_{18}$-Alkyl, $C_1$-$C_{18}$-Alkoxy, Halogen, Cyano oder $C_1$-$C_{20}$-Alkoxy- oder $C_3$-$C_6$-Cycloalkoxycarbonyl steht, wobei beim Vorliegen mehrerer Reste $R^3$ diese gleich oder verschieden sein können,
- X NH, O oder S bedeutet,
- m für die Zahl 1 oder 2 steht und
- n eine ganze Zahl von 1 bis 5 bezeichnet,

mit Ausnahme des Falles, daß gleichzeitig $R^1$ Wasserstoff bedeutet, $R^2$ Cyano oder Ethoxycarbonyl bezeichnet, $R^3$ für Wasserstoff, einen p-Methoxyrest, einen m-Methylrest oder einen m-Chlorsubstituenten steht und X NH bedeutet.

Insbesondere betrifft die vorliegende Erfindung solche Aminovinyl-substituierten Heterocyclen I, bei denen X NH bedeutet.

Einige der substituierten Heterocyclen I sind aus der Literaturstelle J. Heterocycl. Chem. 23, S. 1443-1449 (1986) (2) bekannt, Hinweise auf mögliche Anwendungen werden nicht gegeben. Ein anderer Teil der substituierten Heterocyclen I wird in der Literaturstelle J. Heterocycl. Chem. 28 (2), S. 485-487 (1991) (3) beschrieben, auch dort werden keine Hinweise auf mögliche Anwendungen gegeben.

Die Herstellung der Verbindungen I kann nach allen hierfür üblichen Methoden erfolgen. Es hat sich jedoch als besonders vorteilhaft herausgestellt, hierzu Heterocyclen-Derivate der allgemeinen Formel II

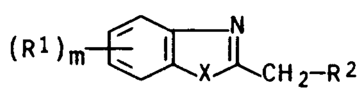

II

mit einem aromatischen Amin der allgemeinen Formel III

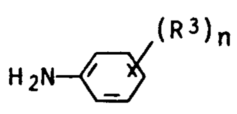

III

und einem Trialkylorthoformiat umzusetzen.

Als geeignete Heterocyclen-Derivate II sind z.B. Benzimidazol-2-acetonitril, Benzimidazol-2-essigsäuremethylester und Benzimidazol-2-essigsäureethylester sowie die entsprechenden Benzoxazol- und Benzthiazol-Derivate zu nennen.

Als Trialkylorthoformiate haben sich Trimethylorthoformiat und vor allem Triethylorthoformiat bewährt.

Die Umsetzung wird zweckmäßigerweise in einem geeigneten polaren organischen Lösungsmittel wie einem Alkohol, z.B. n-Propanol, n-Butanol, Ethylenglykol, Diethylenglykol, Ethylenglykolmonomethylether, Cyclohexanol oder ähnlichen Verbindungen, vorgenommen. Als Lösungsmittel eignen sich auch Carbonsäureamide wie Dimethylformiat oder überschüssiges Trialkylorthoformiat. Bilden die verwendeten Ausgangsverbindungen bereits eine flüssige Mischung, kann auf ein zusätzliches Lösungsmittel verzichtet werden.

Man führt die Umsetzung in der Regel bei Temperaturen von 70 bis 180 °C, vorzugsweise 100 bis 150 °C, und bei Normaldruck durch. Die drei Reaktionspartner werden zweckmäßigerweise im äquimolaren oder annähernd äquimolaren Verhältnis eingesetzt, wenn das Trialkylorthoformiat nicht zusätzlich als Lösungsmittel dient; leichte Überschüsse des einen doer anderen Reaktionspartners, etwa bis zu 15 %,

können dabei in Kauf genommen werden.

Als Katalysatoren für die Umsetzung können bei sehr langen Reaktionszeiten gewünschtenfalls zusätzlich Lewis-Säuren wie $AlCl_3$, $ZrCl_4$, $TiCl_4$ oder vor allem $ZnCl_2$ in den hierfür üblichen Mengen verwendet werden.

Will man Aminovinyl-substituierte Heterocyclen I mit Cycloalkylresten oder längerkettigen Alkylresten in der Alkoxycarbonylgruppe von $R^2$ herstellen, empfiehlt es sich, von Verbindungen I mit niederen Alkoxycarbonylgruppen für $R^2$, beispielsweise den Methoxycarbonyl- oder Ethoxycarbonyl-Derivaten, auszugehen und diese mit den entsprechenden Cycloalkanolen oder längerkettigen Alkanolen nach den üblichen Methoden umzuestern.

Die erfindungsgemäß zu verwendenden Aminovinyl-substituierten Heterocyclen I eignen sich in hervorragender Weise zum Stabilisieren von organischem Material gegen die Einwirkung von Licht, Sauerstoff und Wärme. Sie sind auch wirksam als Metalldesaktivator. Sie werden dem zu stabilisierenden organischen Material in einer Konzentration von 0,01 bis 5 Gew.%, vorzugsweise von 0,02 bis 2 Gew.%, bezogen auf das organische Material, vor, während oder nach seiner Herstellung zugesetzt.

Unter organischem Material sind beispielsweise kosmetische Präparate wie Salben und Lotionen, Arzneimittelformulierungen wie Pillen und Zäpfchen oder Vorprodukte für Kunststoffe und Lacke, insbesondere jedoch Kunststoff und Lacke selbst, zu verstehen.

Gegenstand der vorliegenden Erfindung ist außerdem gegen die Einwirkung von Licht, Sauerstoff und Wärme stabilisiertes organisches Material, insbesondere Kunststoff und Lacke, welches die Verbindungen I in den oben angegebenen Konzentrationen enthält.

Zur Vermischung der erfindungsgemäß zu verwendenden Verbindungen I vor allem mit Kunststoffen können alle bekannten Vorrichtungen und Methoden zum Einmischen von Stabilisierungsmitteln oder anderen Zusätzen in Polymere angewandt werden.

Das durch die erfindungsgemäß zu verwendenden Verbindungen I stabilisierte organische Material kann gegebenenfalls noch weitere Additive enthalten, z.B. Antioxidantien, Lichtstabilisierungsmittel, Metalldesaktivatoren, antistatische Mittel, flammhemmende Mittel, Pigmente und Füllstoffe.

Antioxidantien und Lichtstabilisatoren, die neben den erfindungsgemäßen Verbindungen zugesetzt werden können, sind z.B. Verbindungen auf der Basis sterisch gehinderter Phenole oder Schwefel oder Phosphor enthaltende Costabilisatoren.

Als derartige phenolische Antioxidationsmittel seien beispielsweise 2,6-Di-tert.-butyl-4-methylphenol, n-Octadecyl-β-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-isocyanurat, 1,3,5-Tris-[β-(3,5-di-tert.-butyl-4-hydroxyphenyl)-propionylethyl]isocyanurat, 1,3,5-Tris-(2,6-dimethyl-3-hydroxy-4-tert.-butylbenzyl)-isocyanuratund Pentaerythrit-tetrakis-[β-(3,5-di-tert.-butyl-4-hydroxy-phenyl)-propionat] erwähnt.

Als phosphorhaltige Antioxidantien kommen beispielsweise Tris-(nonylphenyl)-phosphit, Distearylpentaerythritdiphosphit, Tris-(2,4-di-tert.-butyl-phenyl)-phosphit, Tris-(2-tert.-butyl-4-methylphenyl)-phosphit, Bis-(2,4-di-tert.-butylphenyl)-pentaerythritdiphosphit und Tetrakis-(2,4-di-tert.-butylphenyl)-4,4'-biphenylendiphosphit in Betracht.

Als Schwefel enthaltende Antioxidationsmittel seien beispielsweise Dilaurylthiodipropionat, Dimyristylthiodipropionat, Distearylthiodipropionat, Pentaerythrittetrakis-(β-laurylthiopropionat) und Pentaerythrittetrakis-(β-hexylthiopropionat) genannt.

Weitere Antioxidantien und Lichtstabilisatoren, die zusammen mit den Verbindungen I verwendet werden können, sind z.B. 2-(2'-Hydroxyphenyl)benztriazole, 2-Hydroxybenzophenone, Arylester von Hydroxybenzoesäuren, α-Cyanozimtsäurederivate, Benzimidazolcarbonsäureanilide, Nickelverbindungen oder Oxalsäuredianilide.

Eine besonders gute Stabilisierung erhält man, wenn zu den Verbindungen der allgemeinen Formeln I noch mindestens einen Lichtstabilisator aus der Verbindungsklasse der sterisch gehinderten Amine in üblicher Konzentration zusetzt.

Als sterisch gehinderte Amine kommen z.B. in Betracht: Bis-(2,2,6,6-tetramethylpiperidyl)-sebacat, Bis-(1,2,2,6,6-pentamethylpiperidyl)-sebacat, das Kondensationsprodukt von 1-Hydroxyethyl-2,2,6,6-tetramethyl-4-hydroxypiperidin und Bernsteinsäure, das Kondensationsprodukt von N,N'-(2,2,6,6-Tetramethylpiperidyl)-hexamethylendiamin und 4-tert.-Octylamino-2,6-dichlor-1,3,5-s-triazin, Tris-(2,2,6,6-Tetramethylpiperidyl)-nitrolotriacetat, Tetrakis-(2,2,6,6-tetramethyl-4-piperidyl)-1,2,3,4-butan-tetracarbonsäure, 1,1'-(1,2-Ethandiyl)-bis-(3,3,5,5-tetramethylpiperazinon), die Kondensationsprodukte von 4-Amino-2,2,6,6-tetramethylpiperidinen und Tetramethylolacetylendiharnstoffen.

Als Kunststoffe, die durch die erfindungsgemäß zu verwendenden Verbindungen I stabilisert werden können, seien beispielsweise genannt:

Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol;
Copolymere von Styrol oder $\alpha$-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methylacrylat, Acrylnitril-Butadien-Styrol (ABS) oder Methylmethacrylat-Butadien-Styrol (MBS);
halogenhaltige Polymere, wie z.B. Polyvinylchlorid, Polyvinylfluorid, Polyvinylidenfluorid sowie deren Copolymere;
Polymere, die sich von $\alpha,\beta$-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, z.B. Polyvinylalkohol und Polyvinylacetat;
Polyurethane, Polyamide, Polyharnstoffe, Polyester, Polycarbonate, Polysulfone, Polyethersulfone und Polyetherketone.

Weiterhin können mit den erfindungsgemäß zu verwendenden Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die Verbindungen I können in fester oder gelöster Form dem Lack zugesetzt werden. Ihre gute Löslichkeit in Lacksystemen ist dabei von besonderem Vorteil.

Auch bei der erfindungsgemäßen Verwendung als Stabilisatoren in Lacken können die bereits aufgeführten zusätzlichen Additive, insbesondere Antioxidantien und Lichtstabilisatoren, mitverwendet werden.

Die erfindungsgemäß zu verwendenden Verbindungen I eignen sich in besonderem Maße zur Stabilisierung von Polystyrol, Copolymeren aus Styrol-Acrylnitril (SAN) und Acrylnitril-Butadien-Styrol (ABS), Polyurethanen, Polyamiden, Polyolefinen sowie von Lacken.

Eine ganz besonders gute Stabilisierung von Polyurethanen erhält man, wenn das Polyurethan mit einem Gemisch aus mindestens einer Verbindung der allgemeinen Formel I, mindestens einem der oben genannten Antioxidantien und mindestens einer der sterisch gehinderten Aminverbindungen stabilisiert wird.

Weiterhin eignen sich die erfindungsgemäß zu verwendenden Aminovinylsubstituierten Heterocyclen I auch als Lichtschutzmittel in kosmetischen Zubereitungen, also insbesondere zum vorsorglichen Schutz der menschlichen Haut vor der schädigenden Einwirkung von Licht, speziell Sonnenlicht, aber auch künstlichem Licht, welches hohe UV-Anteile aufweist. Unter organischen Materialien ist im weitesten Sinne somit auch die menschliche Haut zu verstehen. Die kosmetischen Zubereitungen als solche werden zugleich natürlich auch stabilisiert, um möglichst lange wirksam zu bleiben.

Demgemäß sind auch Gegenstand der vorliegenden Erfindung kosmetische Zubereitungen, welche 0,1 bis 10 Gew.%, vorzugsweise 1 bis 7 Gew.%, bezogen auf die Menge der kosmetischen Zubereitung, eines oder mehrerer Aminovinyl-substituierter Heterocyclen I als Lichtschutzmittel enthalten. Derartige kosmetische Zubereitungen sind beispielsweise Sonnenschutzpräparate in flüssiger, fester oder pastöser Form wie Cremes, Lotionen, Aerosol-Schaumcremes, Gele, Öle, Fettstifte, Puder oder Sprays.

Die Verbindungen I werden in den kosmetischen Zubereitungen in den üblichen Trägermedien oder Verdünnungsmitteln eingesetzt, beispielsweise als Lösung in einem kosmetischen Öl. Übliche Ölkomponenten in der Kosmetik sind beispielsweise Paraffinöl, Glycerylstearat, Isopropylmyristat, Diisopropyladipat, 2-Ethylhexansäure-cetylstearylester, hydriertes Polyisobuten, Vaseline, Caprylsäure/Caprinsäure-Triglyceride, mikrokristallines Wachs, Lanolin und Stearinsäure. Von besonderem Vorteil ist die gute Löslichkeit der Verbindungen I in diesen Ölkomponenten.

Die Verbindungen I zeichnen sich durch ein besonders hohes Absorptionsvermögen im Bereich der UV-A-Strahlung aus und besitzen eine hohe Photostabilität. Sie können auch vorteilhaft in Kombination mit üblichen UV-B-Filtersubstanzen wie

- 2-Hydroxy-4-methoxybenzophenon
- 2-Hydroxy-4-methoxybenzophenon-5-sulfonsäure
- mit 25 mol Ethylenoxid umgesetzter p-Aminobenzoesäureethylester
- p-Methoxyzimtsäure-2-ethylhexylester
- p-(N,N-Dimethylamino)benzoesäure-2-ethylhexylester
- 2-Phenylbenzimidazol-5-sulfonsäure
- 3-(4-Methylbenzyliden)campher

- 2,4,6-Trianilino-p-(carbo-2'-ethylhexyl-1'-oxy)-1,3,5-triazin

verwendet werden. Diese können in den hierfür üblichen Mengen mitverwendet werden, wobei sogar eine Potenzierung der Lichtschutzwirkung durch einen synergistischen Effekt auftreten kann.

In besonderem Maße eignen sich solche Aminovinyl-substituierten Heterocyclen I als Lichtschutzmittel in kosmetischen Zubereitungen, welche als Rest $R^2$ eine Alkoxycarbonylgruppe mit einem langkettigen Alkylrest tragen. Besonders vorteilhaft sind hierbei geradkettige oder in geringem Maße verzweigte $C_6$-$C_{20}$-Alkylreste, vor allem $C_8$-$C_{18}$-Alkylreste.

Die erfindungsgemäß zu verwendenden Verbindungen I zeichnen sich durch eine gute Verträglichkeit mit den üblichen Kunststoffarten und durch eine gute Löslichkeit in den üblichen Lacksystemen und in den üblichen kosmetischen Ölen aus. Sie haben in der Regel keine oder nur eine sehr geringe Eigenfarbe, sind bei den üblichen Kunststoff- und Lack-Verarbeitungstemperaturen stabil und nicht flüchtig und bewirken vor allen Dingen eine lange Schutzdauer der mit ihnen behandelten organischen Materialien.

Herstellungsbeispiele

Beispiel 1

23,6 g (0,15 mol) Benzimidazol-2-acetonitril, 17,6 g (0,16 mol) p-Toluidin sowie 25,2 g (0,17 mol) Triethylorthoformiat wurden in 60 ml Ethylenglykol 2 h auf 110 °C erhitzt. Es wurde langsam Ethanol abdestilliert, wobei die Temperatur auf ca. 150 °C anstieg. Nach Abkühlung auf 80 °C wurden 120 ml Methanol zugesetzt und es wurde auf Raumtemperatur abgekühlt. Der Niederschlag wurde abfiltriert und mit Methanol gewaschen. Anschließend wurde aus n-Butanol unter Zusatz von Tierkohle und Bleicherde umkristallisiert. Man isolierte 28,6 g Produkt (entsprechend einer Ausbeute von 70 %) in Form blaßgelber Kristalle vom Schmelzpunkt 247-48 °C.

| Analyse: | | | |
|---|---|---|---|
| | C | H | N |
| ber.: | 74,43 | 5,14 | 20,42 |
| gef.: | 74,0 | 5,3 | 20,5 |

Die spektroskopischen Daten sind in Tabelle 1 angegeben.

Beispiel 2

24,5 g (0,12 mol) Benzimidazol-2-essigsäureethylester, 11,6 g (0,125 mol) Anilin sowie 19,6 g (0,132 mol) Triethylorthoformiat wurden in 90 ml Ethylenglykol zunächst auf 100 °C erhitzt. Das bei der Umsetzung entstandene Ethanol wurde über den Zeitraum von 1 bis 2 h abdestilliert, wobei die Temperatur auf ca. 140 °C anstieg. Danach setzte man bei 70 °C 100 ml Ethanol zu, kühlte auf Raumtemperatur ab und filtrierte den gebildeten Niederschlag ab. Das gelbbräunliche Rohprodukt wurde aus Ethanol unter Zusatz von Tierkohle umkristallisiert. 26,3 g Produkt (entsprechend einer Ausbeute von 71,5 %) konnten in Form farbloser Kristalle vom Schmelzpunkt 162-63 °C isoliert werden.

| Analyse: | | | | |
|---|---|---|---|---|
| | C | H | N | O |
| ber.: | 70,36 | 5,54 | 13,68 | 10,6 |
| gef.: | 70,40 | 5,6 | 13,6 | 10,4 |

Die spektroskopischen Daten sind in Tabelle 1 angegeben.

Beispiele 3 bis 22

Analog zu Beispiel 1 bzw. 2 wurden unter Verwendung der entsprechenden aromatischen Amine III die in Tabelle 1 aufgeführten Produkte aus Benzimidazol-2-acetonitril (s. Beispiel 1) bzw. Benzimidazol-2-essigsäureethylester (s. Beispiel 2) und Triethylorthoformiat hergestellt. Die Schmelzpunkte und die spektroskopischen Daten der Produkte sind ebenfalls in Tabelle 1 angegeben.

Beispiel 23

8,0 g (0,026 mol) der Verbindung aus Beispiel 2 wurden in 40 g (0,25 mol) iso-Decanol unter Zusatz von 1 g $Na_2CO_3$ suspendiert und unter Durchleiten von Stickstoff 19 h auf 140 bis 145°C erhitzt. Danach wurde heiß vom anorganischen Salz abfiltriert und der Alkohol im Vakuum abdestilliert. Der Rückstand wurde mit Methanol verrieben und abfiltriert. Man erhielt 8,1 g Produkt (entsprechend einer Ausbeute von 74,3 %) in Form farbloser Kristalle vom Schmp. 80-83°C.

| Analyse: | | | | |
|---|---|---|---|---|
| | C | H | N | O |
| ber.: | 74,43 | 7,93 | 10,02 | 7,73 |
| gef.: | 74,1 | 7,9 | 10,0 | 8,2 |

Die spektroskopischen Daten sind in Tabelle 1 angegeben.

Beispiele 24 bis 26

Analog zu Beispiel 23 wurden unter Verwendung der Produkte aus den Beispielen 14, 18 und 22 sowie des entsprechenden langkettigen Alkohols, nämlich iso-Decanol oder 2-Ethylhexanol, die in Tabelle 1 aufgeführten Verbindungen hergestellt. Die Schmelzpunkte und die spektroskopischen Daten dieser Verbindungen sind dort ebenfalls angegeben.

Beispiel 27

Eine Mischung aus 24,6 g (0,12 mol) Benzoxazol-2-essigsäureethylester, 11,6 g (0,125 mol) Anilin, 19,6 g (0,132 mol) Triethylorthoformiat und 0,5 g Zinkchlorid wurde zunächst auf 100°C erhitzt. Das bei der Umsetzung entstandene Ethanol wurde über den Zeitraum von 1 Stunde abdestilliert, wobei die Temperatur auf ca. 120°C anstieg. Danach setzte man bei 70°C 100 ml Ligroin (Siedebereich 70 bis 80°C) zu, kühlte auf Raumtemperatur ab und filtrierte den gebildeten Niederschlag ab. Das erhaltene Rohprodukt wurde aus Ethanol unter Zusatz von Tierkohle umkristallisiert. Man erhielt das Produkt in Form farbloser Kristalle vom Schmelzpunkt 95 bis 96°C in einer Ausbeute. von 88 %.

Die spektroskopischen Daten sind in Tabelle 1 angegeben.

Beispiele 28 bis 32

Die Herstellung der Beispiele 28, 29, 31 und 32 erfolgte analog zu Beispiel 27. Beispiel 30 wurde aus dem Produkt von Beispiel 29 in Analogie zu Beispiel 23 unter Verwendung von 2-Ethylhexanol hergestellt. Die Strukturen, die Schmelzpunkte und die spektroskopischen Daten der Produkte sind in Tabelle 1 angegeben.

Tabelle 1

Struktur, Schmelzpunkt und spektroskopische Daten der hergestellten Aminovinyl-substituierten Heterocyclen I

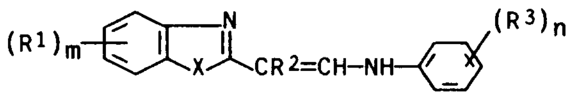

(I)

| Bsp. Nr. | Struktur $R^2$ | $R^3$ | n | Schmelzpunkt [°C] | UV-Daten ($CH_3OH$) $\lambda_{max}$ [nm] | ε |
|---|---|---|---|---|---|---|
| X = NH (Benzimidazole) | | | | | | |
| 1 | CN | 4-$CH_3$ | 1 | 247-48 | 359 | 35300 |
| 2 | $COOC_2H_5$ | H | 1 | 162-63 | 355 | 39500 |
| 3 | CN | 4-$OC_2H_5$ | 1 | 225 | 360 | 34300 |
| 4 | CN | 4-$COOC_2H_5$ | 1 | 258 | 367 | 48600 |
| 5 | CN | 2-$OCH_3$<br>5-Cl | 2 | 260 | 368 | 33600 |
| 6 | CN | 3-$CH_3$<br>4-$CH_3$ | 2 | 251 | 359 | 38300 |
| 7 | CN | 3-$CH_3$<br>5-$CH_3$ | 2 | 269-71 | 359 | 35500 |
| 8 | CN | 2-$CH_3$<br>6-$CH_3$ | 2 | 273 | 331 | 30000 |
| 9 | CN | 2-$COOCH_3$ | 1 | 225 | 365 | 27300 |
| 10 | CN | 2-$CH_3$ | 1 | 248 | 360 | 34700 |
| 11 | CN | 3-$CH_3$ | 1 | 228 | 357 | 35300 |
| 12 | CN | 4-CN | 1 | 313 | 366 | 28500 |

Tabelle 1 (Fortsetzung)

| Bsp. Nr. | Struktur $R^2$ | $R^3$ | n | Schmelzpunkt [°C] | UV-Daten ($CH_3OH$) $\lambda_{max}$ [nm] | ε |
|---|---|---|---|---|---|---|
| 13 | CN | 2-$CH_3$ 4-$OCH_3$ | 2 | 204-06 | 362 | 32000 |
| 14 | $COOC_2H_5$ | 4-$CH_3$ | 1 | 201 | 358 | 21700 |
| 15 | $COOC_2H_5$ | 2-$CH_3$ | 1 | 148 | 359 | 34300 |
| 16 | $COOC_2H_5$ | 4-$OC_2H_5$ | 1 | 180-82 | 360 | 32500 |
| 17 | $COOC_2H_5$ | 3-$CH_3$ | 1 | 147 | 356 | 31900 |
| 18 | $COOC_2H_5$ | 4-$COOC_2H_5$ | 1 | 183 | 369 | 43500 |
| 19 | $COOC_2H_5$ | 3-$CH_3$ 5-$CH_3$ | 2 | 172 | 362 | 36500 |
| 20 | $COOC_2H_5$ | 3-$CH_3$ 4-$CH_3$ | 2 | 171 | 358 | 35200 |
| 21 | $COOC_2H_5$ | 3-$OCH_3$ 4-$OCH_3$ | 2 | 162 | 372 | 32600 |
| 22 | $COOC_2H_5$ | 2-$CH_3$ 4-$OCH_3$ | 2 | 206 | 363 | 30000 |
| 23 | COO-iso-$C_{10}H_{21}$ | H | 1 | 80-83 | 356 | 33600 |
| 24 | COO-iso-$C_{10}H_{21}$ | 4-$CH_3$ | 1 | 80-82 | 358 | 32100 |
| 25 | COO-$C_8H_{17}$ | 4-COO-$C_8H_{17}$ | 1 | 60 | 370 | 48900 |
| 26 | COO-$C_8H_{17}$ | 2-$CH_3$ 4-$OCH_3$ | 2 | 76-78 | 362 | 33000 |
| X = O (Benzoxazole) | | | | | | |
| 27 | $COOC_2H_5$ | H | 1 | 95-96 | 351 | 32500 |
| 28 | $COOC_2H_5$ | 4-$OCH_3$ | 1 | 95-96 | 355 | 31600 |
| 29 | $COOC_2H_5$ | 4-$COOC_2H_5$ | 1 | 134-36 | 363 | 44000 |
| 30 | COO-$C_8H_{17}$ | 4-COO-$C_8H_{17}$ | 1 | öl | 363 | 41600 |
| 31 | $COOC_2H_5$ | 4-$CH_3$ | 1 | 123 | 353 | 30800 |
| 32 | $COOC_2H_5$ | 4-COO-$C_8H_{17}$ | 1 | 63-65 | 363 | 42600 |

Anmerkungen:

In allen Beispielen ist $R^1$ = H und m = 1.

Unter $C_8H_{17}$ in den Beispielen 25, 26, 30 und 32 ist der 2-Ethylhexylrest zu verstehen; bei den Beispielen 25 und 30 trat zusätzlich eine Umesterung an der 4-$COOC_2H_5$-Gruppe des Phenylkerns auf.

Anwendungsbeispiele

Zur Herstellung von Belichtungsproben aus Polyurethan wurde eine Mischung aus

100 g einer Polyolkomponente der Zusammensetzung

41,9 g eines Polyetherols der OH-Zahl 29 mit ca. 84 % primären Hydroxylgruppen, erhalten durch Addition von Propylenoxid und Ethylenoxid an Polypropylenglykol,

42,5 g eines Polyetherols der OH-Zahl 27 mit ca. 88 % primären Hydroxylgruppen, erhalten durch

Addition von Propylenoxid und Ethylenoxid an Trimethylolpropan, und
8,1 g 1,4-Butandiol,
1,7 g einer 25 gew.%igen Lösung von 1,4-Diazabicyclo[2.2.2]octan in 1,4-Butandiol,
0,02 g Dibutylzinndilaurat,
0,1 g eines handelsüblichen Siliconstabilisators,
5,5 g Fluortrichlormethan und
0,2 g Wasser

mit 0,5 g des Produktes aus Beispiel 2 bzw. zum Vergleich mit derselben Menge eines Mittels des Standes der Technik,
0,5 g Bis(1,2,2,6,6-pentamethylpiperidyl)sebacat sowie
0,25 g eines Antioxidans-Gemisches aus 9 Gew.% α-Tocopherol und 91 Gew.% Tris(nonylphenyl)phosphit versetzt
und mit 48,5 g eines 23 Gew.% Isocyanatgruppen enthaltenden Prepolymeren, hergestellt aus
87,2 Gew.% 4,4'-Diphenylmethandiisocyanat,
4,8 Gew.% eines Polyetherols der OH-Zahl 250, erhalten durch Addition von Propylenoxid an Propylenglykol, und
8,0 Gew.% Dipropylenglykol

bei 25 °C Komponenten- und Werkzeugtemperatur zu Prüfplatten verschäumt.

Die Prüfplatten wurden im Xenotest® 450 der Firma Hanau belichtet und an den Proben wurde der Yellowness Index (YI) gemäß Annual Book of ASTM Standards D 1925-70 (Reapproved 1977) als Maß für den Vergilbungsgrad bestimmt. Die Ergebnisse sind in Tabelle 2 zusammengestellt.

Tabelle 2

YI-Werte von Polyurethan-Proben

| | nach 48 h | nach 96 h |
|---|---|---|
| erfindungsgemäß: | | |
| mit Substanz aus Beispiel 2 | 11,5 | 14,0 |
| mit Substanz aus Beispiel 23 | 12,4 | 13,4 |
| zum Vergleich: | | |
| ohne jeglichen Stabilisator | 41,4 | 53,1 |
| mit Mittel des Standes der Technik* | 13,1 | 15,8 |

* Verbindung

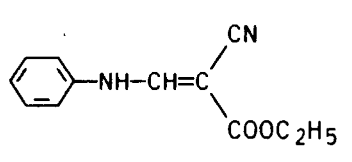

gemäß US-A 3 079 366 (1)

Als anwendungstechnische Prüfungen für eine kosmetische Anwendung wurden Löslichkeitsversuche durchgeführt sowie die Photostabilitäten in kosmetischen Ölen untersucht.

Löslichkeitsversuche

Löslichkeitsversuche in Paraffinöl, 2-Ethylhexansäure-cetylstearylester, Caprylsäure/Caprinsäure-Triglyceriden und $C_{12}$-$C_{15}$-Alkoholbenzoaten ergaben bei Beispiel 25 und 30 eine Löslichkeit von mehr als 10 Gew.-%.

Photostabilität

Die Photostabilitätsmessungen wurden in einem Gemisch aus $C_{12}$-$C_{15}$-Alkoholbenzoaten als Lösungsmittel durchgeführt. Dazu wurde eine Lösung der in Tabelle 3 aufgeführten Verbindungen der Konzentrationen 10 mg/1000 ml mit einer Xenonlampe bestrahlt und als Maß für die Zerstörung die Abnahme der Extinktion der längstwelligen Absorptionsbande in Abhängigkeit von der Bestrahlungszeit registriert.

Die Ergebnisse sind in der Tabelle 3 wiedergegeben.

Tabelle 3

| Beispiel Nr. | Extinktion nach 0 h | Extinktion nach 6 h |
|---|---|---|
| 25 | 0,805 (100 %) | 0,788 (97,9 %) |
| 30 | 0,757 (100 %) | 0,719 (95,0 %) |

**Patentansprüche**

**1.** Verwendung von Aminovinyl-substituierten Heterocyclen der allgemeinen Formel I

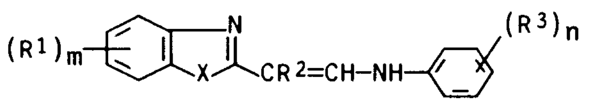

I

in der

- $R^1$ Wasserstoff, $C_1$-$C_4$-Alkyl, $C_1$-$C_4$-Alkoxy oder Halogen bezeichnet, wobei beim Vorliegen mehrerer Reste $R^1$ diese gleich oder verschieden sein können,
- $R^2$ Cyano oder $C_1$-$C_{20}$-Alkoxy- oder $C_3$-$C_6$-Cycloalkoxycarbonyl bedeutet,
- $R^3$ für Wasserstoff, $C_1$-$C_{18}$-Alkyl, $C_1$-$C_{18}$-Alkoxy, Halogen, Cyano oder $C_1$-$C_{20}$-Alkoxy- oder $C_3$-$C_6$-Cycloalkoxycarbonyl steht, wobei beim Vorliegen mehrerer Reste $R^3$ diese gleich oder verschieden sein können,
- X NH, O oder S bedeutet,
- m für die Zahl 1 oder 2 steht und
- n eine ganze Zahl von 1 bis 5 bezeichnet,

als Stabilisatoren für organische Materialien.

**2.** Verwendung von Aminovinyl-substituierten Heterocyclen I nach Anspruch 1, bei denen

- $R^1$ Wasserstoff, Methyl, Methoxy oder Chlor bezeichnet,
- $R^2$ Cyano oder $C_1$-$C_{20}$-Alkoxycarbonyl bedeutet,
- $R^3$ für Wasserstoff, $C_1$-$C_4$-Alkyl, $C_1$-$C_4$-Alkoxy, Chlor, Cyano oder $C_1$-$C_{12}$-Alkoxycarbonyl steht,
- X NH bedeutet,
- m für die Zahl 1 oder 2 steht und
- n die Zahl 1 oder 2 bezeichnet.

**3.** Verwendung von Aminovinyl-substituierten Heterocyclen I nach Anspruch 1 oder 2, bei denen

- $R^1$ Wasserstoff bezeichnet,
- $R^2$ $C_1$-$C_{20}$-Alkoxycarbonyl bedeutet,
- $R^3$ für Wasserstoff, Methyl, Ethyl, Methoxy, Ethoxy, Chlor, Cyano oder $C_1$-$C_8$-Alkoxycarbonyl steht,
- X NH bedeutet und
- n die Zahl 1 oder 2 bezeichnet.

**4.** Aminovinyl-substituierte Heterocyclen der allgemeinen Formel I

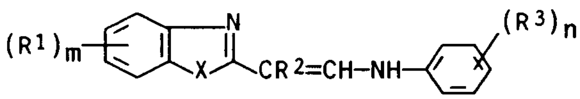

I

in der

- $R^1$ Wasserstoff, $C_1$-$C_4$-Alkyl, $C_1$-$C_4$-Alkoxy oder Halogen bezeichnet, wobei beim Vorliegen mehrerer Reste $R^1$ diese gleich oder verschieden sein können,
- $R^2$ Cyano oder $C_1$-$C_{20}$-Alkoxy- oder $C_3$-$C_6$-Cycloalkoxycarbonyl bedeutet,
- $R^3$ für Wasserstoff, $C_1$-$C_{18}$-Alkyl, $C_1$-$C_{18}$-Alkoxy, Halogen, Cyano oder $C_1$-$C_{20}$-Alkoxy- oder $C_3$-$C_6$-Cycloalkoxycarbonyl steht, wobei beim Vorliegen mehrerer Reste $R^3$ diese gleich oder verschieden sein können,
- X NH, O oder S bedeutet,
- m für die Zahl 1 oder 2 steht und
- n eine ganze Zahl von 1 bis 5 bezeichnet,

mit Ausnahme des Falles, daß gleichzeitig $R^1$ Wasserstoff bedeutet, $R^2$ Cyano bezeichnet, $R^3$ für Wasserstoff oder einen p-Methoxyrest steht und X NH bedeutet.

**5.** Verfahren zur Herstellung von Aminovinyl-substituierten Heterocyclen I gemäß Anspruch 4, dadurch gekennzeichnet, daß man Heterocyclen-Derivate der allgemeinen Formel II

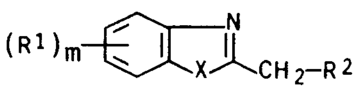

II

mit aromatischen Aminen der allgemeinen Formel III

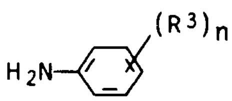

III

und einem Trialkylorthoformiat umsetzt.

**6.** Verwendung von Aminovinyl-substituierten Heterocyclen I nach den Ansprüchen 1 bis 3 als Stabilisatoren für Kunststoffe und Lacke.

**7.** Verwendung von Aminovinyl-substituierten Heterocyclen I nach den Ansprüchen 1 bis 3 als Lichtschutzmittel in kosmetischen Zubereitungen.

**8.** Gegen die Einwirkung von Licht, Sauerstoff und Wärme stabilisierte organische Materialien, enthaltend 0,01 bis 5 Gew.%, bezogen auf die Menge des organischen Materials, eines oder mehrerer Aminovinyl-substituierter Heterocyclen I gemäß den Ansprüchen 1 bis 3.

**9.** Gegen die Einwirkung von Licht, Sauerstoff und Wärme stabilisierte Kunststoffe und Lacke, enthaltend 0,01 bis 5 Gew.%, bezogen auf die Menge des Kunststoffes bzw. Lackes, eines oder mehrerer Aminovinyl-substituierter Heterocyclen I gemäß den Ansprüchen 1 bis 3.

**10.** Kosmetische Zubereitungen, enthaltend 0,1 bis 10 Gew.%, bezogen auf die Menge der kosmetischen Zubereitung, eines oder mehrerer Aminovinyl-substituierter Heterocyclen I gemäß den Ansprüchen 1 bis 3 als Lichtschutzmittel.

**11.** Verfahren zum Stabilisieren von organischen Materialien, dadurch gekennzeichnet, daß man hierzu Aminovinyl-substituierte Heterocyclen I gemäß den Ansprüchen 1 bis 3 verwendet.

**12.** Verfahren zum Stabilisieren von Kunststoffen und Lacken, dadurch gekennzeichnet, daß man hierzu Aminovinyl-substituierte Heterocyclen I gemäß den Ansprüchen 1 bis 3 verwendet.

**13.** Verfahren zum Schutz der menschlichen Haut vor der Einwirkung von Licht, dadurch gekennzeichnet, daß man hierzu kosmetische Zubereitungen verwendet, welche Aminovinyl-substituierte Heterocyclen I gemäß den Ansprüchen 1 bis 3 als Lichtschutzmittel enthalten.

Europäisches Patentamt

# EUROPÄISCHER RECHERCHENBERICHT

Nummer der Anmeldung

EP 92 10 3013

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | US-A-3 079 366 (R.J. BOYLE ET AL.)<br>* Anspruch 1 *<br>--- | 1,6,11,<br>12 | C07D235/12<br>C07D235/14<br>C07D263/56<br>C07D277/64<br>C08K5/3447<br>C08K5/353<br>C08K5/47<br>A61K7/42 |
| A | GB-A-984 013 (J.R. GEIGY A.-G.)<br>* Ansprüche *<br>--- | 1,11 | |
| A | DE-A-2 008 464 (CIBA AG)<br>* Ansprüche *<br>--- | 1 | |
| D,X | JOURNAL OF HETEROCYCLIC CHEMISTRY,<br>Bd. 28, Februar 1991,<br>Seiten 485 - 487;<br>T. AOTSUKA ET AL.: 'Ring Transformation ...'<br>* Seite 485, Schema 1, Verbindungen 5a-d; Seite 487, "Experimental" *<br>--- | 4 | |
| A,D | JOURNAL OF HETEROCYCLIC CHEMISTRY,<br>Bd. 23, 1986,<br>Seiten 1443 - 1449;<br>K. TAGAKI ET AL.: 'Synthesis of ...'<br>* Seite 1444, Schema 1, Verbindungen 5 und 6 *<br>--- | 4 | **RECHERCHIERTE SACHGEBIETE (Int. Cl.5)** |
| A | DE-A-1 923 992 (AGFA-GEVAERT AG)<br>* Seite 10 oben, Strukturformel; Seite 11 Mitte, Strukturformel *<br>----- | 4 | C07D<br>C08K<br>A61K |

Der vorliegende Recherchenbericht wurde für alle Patentansprüche erstellt

| Recherchenort | Abschlußdatum der Recherche | Prüfer |
|---|---|---|
| BERLIN | 25 MAI 1992 | Christian Hass |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.....
& : Mitglied der gleichen Patentfamilie, übereinstimmendes Dokument

EPO FORM 1503 03.82 (P0403)